# EUROPÄISCHE PATENTSCHRIFT

(11) **EP 1 250 304 B1**
(45) Veröffentlichungstag und Bekanntmachung des Hinweises auf die Patenterteilung: **07.03.2007**
(21) Anmeldenummer: 00991643.8
(22) Anmeldetag: 27.12.2000
(51) Int. Cl.: C07C 45/80, C07C 45/82, C07C 47/19

(54) **VERFAHREN ZUR DESTILLATIVEN ABTRENNUNG VON FORMALDEHYD AUS POLYOLHALTIGEN REAKTIONSGEMISCHEN DURCH ZUSATZ VON WASSER VOR UND/ODER WÄHREND DER DESTILLATION**
METHOD FOR THE SEPARATION OF FORMALDEHYDE FROM A REACTION MIXTURE CONTAINING POLYOLS BY THE ADDITION OF SOLVENTS BEFORE AND/OR DURING THE DISTILLATION
PROCEDE POUR SEPARER, PAR DISTILLATION, DU FORMALDEHYDE CONTENU DANS DES MELANGES REACTIONNELS RENFERMANT DES POLYOLS, PAR ADDITION D'EAU AVANT ET/OU PENDANT LA DISTILLATION

(30) Priorität: 28.12.1999 DE 19963445
(43) Veröffentlichungstag der Anmeldung: 23.10.2002
(73) Patentinhaber: BASF Aktiengesellschaft, 67056 Ludwigshafen (DE)
(72) Erfinder: DERNBACH, Matthias, 69221 Dossenheim (DE); KRATZ, Detlef, 69121 Heidelberg (DE); STAMMER, Achim, 67251 Freinsheim (DE); SCHULZ, Gerhard, 67069 Ludwigshafen (DE)
(74) Vertreter: Isenbruck, Günter
(86) Internationale Anmeldenummer: PCT/EP2000/013293
(87) Internationale Veröffentlichungsnummer: WO 2001/047854

(56) Entgegenhaltungen:
- WO-A-96/16953
- DD-A- 273 434
- DATABASE WPI Section Ch, Week 199902 Derwent Publications Ltd., London, GB; Class A41, AN 1999-018323 XP002167534 & JP 10 287606 A (MITSUBISHI CHEM CORP), 27. Oktober 1998 (1998-10-27) in der Anmeldung erwähnt

## Beschreibung

Die vorliegende Erfindung bezieht sich auf das Gebiet der industriellen organischen Chemie. Genauer gesagt betrifft die vorliegende Erfindung ein Verfahren zum effektiven Abtrennen von Formaldehyd aus Methylolalkanal-Lösungen. Die Erfindung betrifft weiterhin ein Verfahren zum Hydrieren der derart erhaltenen Methylolalkanale zu Polyolen.

Die Kondensation von Formaldehyd mit CH-aciden höheren Alkanalen zu Methylolalkanalen, im allgemeinen Dimethylol- und Trimethylolalkanalen und Überführung der erhaltenen Verbindungen in Polyole ist ein weit verbreitetes Verfahren in der Chemie. Beispiele für derart erhaltene, wichtige Triole sind Trimethylolpropan, Trimethylolethan und Trimethylolbutan, die zur Herstellung von Lacken, Urethanen und Polyestern ein breites Verwendungsgebiet gefunden haben. Weitere wichtige Verbindungen sind Pentaerythrit, erhältlich durch Kondensation von Formaldehyd und Acetaldehyd, sowie Neopentylglykol aus iso-Butyraldehyd und Formaldehyd. Der vierwertige Alkohol Pentaerythrit wird ebenfalls häufig in der Lackindustrie eingesetzt, hat aber auch eine große Wichtigkeit bei der Herstellung von Sprengstoffen erlangt.

Die erwähnten Polyole können nach verschiedenen Verfahren hergestellt werden. Eine Methode ist das sogenannte Cannizzaro-Verfahren, das noch weiterhin unterteilt wird in das anorganische und das organische Cannizzaro-Verfahren. Bei der anorganischen Variante setzt man einen Überschuß Formaldehyd mit dem entsprechenden Alkanal in Gegenwart von stöchiometrischen Mengen einer anorganischen Base wie NaOH oder Ca(OH)₂ um. Das in der ersten Stufe gebildete Methylolalkanal reagiert in der zweiten Stufe mit dem überschüssigen Formaldehyd in einer Disproportionierungsreaktion zu dem entsprechenden Polyol und dem Formiat der entsprechenden Base, also etwa Natrium- oder Calciumformiat. Der Anfall dieser Salze stellt einen Nachteil dar, da diese schwierig vom Reaktionsprodukt abzutrennen sind und außerdem ein Äquivalent Formaldehyd verloren geht.

Bei dem organischen Cannizzaro-Verfahren wird anstelle einer anorganischen Base ein tertiäres Alkylamin eingesetzt. Dadurch lassen sich höhere Ausbeuten erzielen als mit einer anorganischen Base. Es fällt als unerwünschtes Nebenprodukt Trialkylammoniumformiat an. Somit geht auch hier ein Äquivalent Formaldehyd verloren.

Die Nachteile des Cannizzaro-Verfahrens werden bei dem sogenannten Hydrierverfahren vermieden. Dabei bringt man Formaldehyd mit dem entsprechenden Alkanal in Gegenwart von katalytischen Mengen eines Amins zur Reaktion. Damit wird erreicht, daß die Reaktion auf der Stufe des Methylolalkanals anhält. Nach Abtrennen des Formaldehyds wird das Reaktionsgemisch, das neben dem entsprechenden Alkanal noch geringe Mengen des entsprechenden Triols enthält, einer Hydrierung unterworfen, bei der das gewünschte Polyol erhalten wird.

Verschiedene Varianten dieser Hydrierverfahren sind unter anderem beschrieben in den Anmeldungen DE-A-25 07 461, DE-A-27 02 582, DE-A-27 14 516, DE-A-28 13 201, DE-A-33 40 791 und WO 98/28253.

Ein Hauptproblem des Hydrierverfahrens ist dabei eine wirkungsvolle Abtrennung des im Überschuß eingesetzten Formaldehyds nach der Kondensationsreaktion, vor dem Einsatz des Reaktionsgemisches in die Hydrierung. Eine effektive Abtrennung des Formaldehyds ist allein schon deswegen wünschenswert, weil dieser in die Reaktion zurückgeführt werden kann und damit nicht verloren geht. Prinzipiell wäre es möglich, das Reaktionsgemisch nach der Kondensation direkt zu hydrieren, ohne vorhergehende Abtrennung des Formaldehyds. Dieses würde jedoch in das relativ wertlose Methanol überführt; weiterhin wäre auch ein erhöhter Verbrauch an Wasserstoff sowie eine höhere Belastung des Katalysators die Folge, so daß letztendlich eine ungünstige Bilanz resultieren würde.

Die Literatur beschreibt verschiedene Methoden zur effektiven Abtrennung von Formaldehyd aus den Reaktionsgemischen der Kondensation. Im Zusammenhang mit der vorliegenden Erfindung sind die nachfolgend zitierten Referenzen von Interesse.

In der DD-A-273 434 findet sich die Beschreibung eines Verfahrens zum Abtrennen von überschüssigem Formaldehyd aus Reaktionslösungen, in denen dieser nach dem klassischen Cannizzaro-Verfahren mit höheren Alkanalen zur Reaktion gebracht wird. Dabei wird ein Rohgemisch enthaltend den mehrwertigen Alkohol sowie das im Überschuß eingesetzte Formaldehyd erhalten. Die Abtrennung des Formaldehyds erfolgt durch Zugabe von Methanol, Wasser und/oder Acetonitril und anschließendem azeotropen Abdestillieren des Formaldehyds als komplexes Gemisch mit den zugesetzten Lösungsmitteln und weiteren Komponenten. Im Sumpf verbleibt der mehrwertige Alkohol.

In der Japanischen Patentschrift JP 10287606 (zitiert nach CA 1998/129: 275637) wird ein Verfahren zur Abtrennung von Formaldehyd aus Reaktionsgemischen beschrieben, die aus der Umsetzung dieses Aldehyds mit höheren Aldehyden stammen und das entsprechende Dimethylolalkanal als Produkt enthalten. Die Abtrennung erfolgt durch Destillation an einem Dünnfilmverdampfer. Vor der Destillation wird dem Gemisch Wasser zugegeben, in Mengen von > 4 Gewichtsteilen. Durch die Destillation wird ein Restgehalt an Formaldehyd von 5 % erreicht, bei Drücken von 0,5 bis 1 bar.

Auch die US 4,036,888 beschreibt die Zugabe von Wasser zu Reaktionsgemischen der Umsetzung von Formaldehyd mit höheren Aldehyden und anschließender Destillation. In diesem Fall wird Isobutyraldehyd verwendet, das Produkt ist Hydroxypivalinaldehyd. Durch die Wasserzugabe wird hier die effektive Abtrennung des Isobutyraldehyds erreicht, der im Überschuß eingesetzt wurde. Aus diesem Grund stellt sich das Problem der Abtrennung von Formaldehyd hier nicht, denn dieser liegt nach der Reaktion nur noch in sehr geringen Mengen vor.

Gleiches gilt auch für das Patent US 5,235,118, das die Reaktion von 2-Ethylhexanal mit Formaldehyd zu 2-Ethyl-2-(hydroxymethyl)hexanal zum Gegenstand hat. Auch hier wird der Formaldehyd in unterstöchiometrischen Mengen gegenüber dem 2-Ethylhexanal eingesetzt, es liegt nur noch ein geringer Rest Formaldehyd in der Produktlösung vor. Die Zugabe von Wasser mit anschließender Destillation dient der effektiven Abtrennung von nichtreagiertem 2-Ethylhexanal.

Der vorliegenden Erfindung liegt die Aufgabe zugrunde, ein Verfahren bereitzustellen, das ein effektives Abtrennen des Formaldehyds aus den nach Umsetzung dieses Aldehyds mit höheren Aldehyden erhaltenen Methylolalkanalen ermöglicht. Die Trennung sollte so effektiv sein, daß die nachfolgende Hydrierung des Methylolalkanals wirtschaftlich ist.

Diese Aufgabe wird gelöst durch ein Verfahren zum destillativen Abtrennen von Formaldehyd aus Reaktionslösungen enthaltend ein methyloliertes Alkanal, das aus der Umsetzung von Formaldehyd mit einem Alkanal, das wenigstens ein acides Wasserstoffatom in α-Stellung zur Carbonylfunktion aufweist oder der Umsetzung eines 2-Alkylacroleins oder Acrolein mit Wasser und Formaldehyd erhalten wurde, und wobei diese Umsetzung in Gegenwart katalytischer Mengen eines organischen Amins durchgeführt wurde, dadurch gekennzeichnet, daß der Abtrennen in einer Destillationskolonne erfolgt und vor und/oder während der Destillation 0.1 bis 10 Teile Wasserdampf eingespeist werden.

Diese Aufgabe wird weiterhin gelöst durch ein Verfahren zur Herstellung von Polyolen, dadurch gekennzeichnet, daß ein methyloliertes Alkanal, das gemäß dem oben beschriebenen Verfahren von Formaldehyd befreit wurde, einer an sich bekannten Hydrierung unterworfen wird.

Es wurde festgestellt, daß durch den Zusatz von Wasser in Form in Wasserdampf in den angegebenen Mengen vor und/oder während der Abtrennung des Formaldehyds durch Destillation aus den nach der Kondensation von Formaldehyd mit einem höheren Aldehyd erhaltenen Methylolalkanal eine effektive Trennung erreicht werden kann. Weiterhin kann durch den Zusatz von Wasser die Ausbeute in einer nachfolgenden Hydrierung erhöht werden.

Generell wird es sich bei den Methylolalkanalen um Di - oder auch Trimethylolalkanale handeln. Die Methylolalkanale werden im allgemeinen durch Kondensation der oben genannten Ausgangsverbindungen in Gegenwart von katalytischen Mengen eines organischen Amins hergestellt werden. In dem vorliegenden Verfahren können generell praktisch alle formaldehydhaltigen Aldolisierungsausträge verwendet werden, die nach der erwähnten Kondensationsreaktion hergestellt sind. Besonders vorteilhaft läßt sich das Verfahren bei Aldolisierungsausträgen anwenden, die nach der WO 98/28253 erhalten wurden. Der Inhalt dieser Anmeldung ist durch Referenz in die vorliegende Anmeldung eingeschlossen.

Die Abtrennung des Formaldehyds aus den Aldolisierungsgemischen erfolgt in einer Destillationskolonne.

Geeignete Destillationskolonnen weisen eine Anzahl von Trennstufen von 3 bis 100 auf. Die besten Resultate wurden erzielt mit Trennstufen von 5 bis 60. Diese Trennstufen können mit den üblichen Maßnahmen erreicht werden. Als nicht beschränkende Beispiele seien hier erwähnt Böden, geordnete Packungen oder Füllkörper. Bevorzugterweise werden diese Trennstufen so ausgeführt, daß Verweilzeiten von 1 min bis 300 min, besonders bevorzugt 10 min bis 180 min, erreicht werden.

Die Kolonne wird bei der Destillation mit einer Temperatur von 50°C bis 160°C betrieben. Der gewählte Druck bewegt sich dabei bei Werten von 20 mbar bis 5 bar, bevorzugterweise von 1 bar bis 3 bar, insbesondere 1,5 bis 3 bar. Die Kolonne wird so eingestellt werden, daß ein Rücklaufverhältnis von 100 bis 0, vorzugsweise von 3 bis 0, resultiert. Das aufzutrennende Reaktionsgemisch wird vorzugsweise in der oberen Kolonnenhälfte oder am Kolonnenkopf eingespeist.

Um ein effektives Abtrennen des Formaldehyds zu ermöglichen, wird an einer geeigneten Stelle der Kolonne Wasser in Form von Wasserdampf eingespeist. Die Einspeisung kann an verschiedenen Stellen der Destillationskolonne oder des Sumpfes geschehen, wobei eine Einspeisung mit dem Zulaufstrom oder unterhalb von diesem bevorzugt ist. Besonders bevorzugt ist die Einspeisung in den Kolonnensumpf oder in den Verdampfer. Der Wasserdampf wird so dosiert werden, daß seine Menge 0,1 bis 10 Teile des Zulaufstroms beträgt.

Aufgrund der Temperaturempfindlichkeit der Produktmischung ist es im Zusammenhang mit der vorliegenden Erfindung bevorzugt, die Kolonne mit einem Verdampfer zu betreiben, der eine geringe Wandtemperatur sowie einen kleinen Flüssigkeitsinhalt aufweist. Es hat sich dabei als besonders günstig erwiesen, auf einen Fallfilmverdampfer zurückzugreifen.

In einer bevorzugten Ausführungsform der vorliegenden Erfindung werden der Kolonnensumpf und der Verdampfersumpf so gestaltet, daß die Verweilzeit des Sumpfaustrags, also des von Formaldehyd gereinigten methylolierten Alkanals in Wasser, von 1 min bis 300 min beträgt. Besonders bevorzugte Verweilzeiten betragen von 5 min bis 120 min. Bei diesen derart gewählten Verweilzeiten wird ein Optimum aus der Abtrennung von Formaldehyd und der Vermeidung der Bildung unerwünschter Nebenprodukte erzielt.

Der nach der Reinigung erhaltene Produktstrom, der aus einem Gemisch des methylolierten Alkanals mit Wasser besteht, wird im allgemeinen dem Kolonnensumpf oder, wenn die Destillationsverrichtung einen geteilten Sumpf aufweist, dem hinter dem Verdampfer anfallenden Verdampferumlaufstrom oder dem Verdampfersumpf entnommen werden.

Aufgrund der breiten Kondensationskurve des am Kolonnenkopf austretenden Brüdens ist es vorteilhaft, einen Kondensator mit Flüssigkeitsrückführung zu verwenden. Besonders vorteilhaft ist die Direktkondensation in einem Quench mit gekühltem Flüssigkeitsumlauf.

Das erfindungsgemäße Verfahren kann zum Entfernen von Formaldehyd aus allen Gemischen verwendet werden, die aus dessen Umsetzung mit Alkanalen oder dessen Umsetzung mit Wasser und Acrolein oder 2-Alkylacrolein resultieren. Beispiele für Aldehyde sind aliphatische Aldehyde mit 2 bis 24 C-Atomen, die eine lineare oder verzweigte Kette aufweisen oder auch alicyclische Gruppen enthalten können. Voraussetzung ist lediglich, daß mindestens ein acides Wasserstoffatom in α-Stellung zur Carbonylfunktion vorhanden ist. Dies gilt auch für Aralkylaldehyde, die als Ausgangsmaterial eingesetzt werden können. Im allgemeinen werden solche Aldehyde mit 8 bis 24 C-Atomen, vorzugsweise 8 bis 12 C-Atomen, eingesetzt werden. Beispiele für geeignete Aldehyde sind 3-Ethyl-, 3-n-Propyl-, 3-Isbpropyl-, 3-n-Butyl-, 3-Isobutyl-, 3-sek.-Butyl-, 3-tert.-Butyl-butanal sowie entsprechende -n-pentanale, -n-hexanale, -n-heptanale; 4-Ethyl-, 4-n-Propyl-, 4-Isopropyl-, 4-n-Butyl-, 4-Isobutyl-, 4-sek.-Butyl-, 4-tert.-Butyl-pentanale, -n-hexanale, -n-heptanale; 5-Ethyl-, 5-n-Propyl-, 5-Isopropyl-, 5-n-Butyl-, 5-Isobutyl-, 5-sek.-Butyl-, 5-tert.-Butyl-n-hexanale, -n-heptanale; 3-Methylhexanal, 3-Methyl-heptanal; 4-Methyl-pentanal, 4-Methyl-heptanal, 5-Methyl-hexanal, 5-Methylheptanal; 3,3,5-Trimethyl-n-pentyl-, 3,3-Diethyl-pentyl-, 4,4-Diethylpentyl-, 3,3-Dimethyl-n-butyl-, 3,3-Dimethyl-n-pentyl-, 5,5-Dimethylheptyl-, 3,3-Dimethylheptyl-, 3,3,4-Trimethylpentyl-, 3,4-Dimethylheptyl-, 3,5-Dimethylheptyl-, 4,4-Dimethylheptyl-, 3,3-Diethylhexyl-, 4,4-Dimethylhexyl-, 4,5-Dimethylhexyl-, 3,4-Dimethylhexyl-, 3,5-3,3-Dimethylhexyl-, 3,4-Diethylhexyl-, 3-Methyl-4-ethylpentyl-, 3-Methyl-4-ethylhexyl-, 3,3,4-Trimethylpentyl-, 3,4,4-Trimethylpentyl-, 3,3,4-Trimethylhexyl-, 3,4,4-Trimethylhexyl-, 3,3,4,4-Tetramethylpentylaldehyd; insbesondere C₂- bis C₁₂-n-Alkanale.

Beispiele für Alkylacroleine sind Methacrolein, 2-Ethylacrolein, 2-Propylacrolein und 2-Hydroxymethylacrolein.

Besonders bevorzugt werden Acetaldehyd zur Herstellung von Pentaerythrit, Propionaldehyd zur Herstellung von Trimethylolethan, n-Butyraldehyd zur Herstellung von TMP, n-Pentanal zur Herstellung von Trimethylolbutan und i-Butyraldehyd zur Herstellung von Neopentylglykol als Ausgangsverbindungen eingesetzt.

Der gereinigte Produktstrom wird dann einer Hydrierung unterworfen, um die Alkanalfunktion in eine Alkoholfunktion zu überführen und somit Polyalkohole, im allgemeinen Triole oder auch vierwertige Alkohole oder Diole, herzustellen. Es konnte festgestellt werden, daß die Produktgemische, die aus dem Verfahren nach der vorliegenden Erfindung resultieren und einen höheren Wassergehalt als bisher beschriebene Produktgemische aufweisen, besonders vorteilhafte Resultate bei den Hydrierungen ergeben. Es wurde überraschend gefunden, daß der erhöhte Wassergehalt sich nicht etwa nachteilig auswirkt, sondern das Gegenteil der Fall ist. So wurde festgestellt, daß durch die erhöhte Verdünnung die nachfolgende Hydrierung deutlich selektiver bezüglich des Alkanals wird. Weiterhin hat die erhöhte Verdünnung auch einen positiven Einfluß auf die Lebensdauer des Katalysators, der deutlich langsamer desaktiviert.

Ein Verfahren zur Hydrierung von Methylolalkanalen, die nach dem Verfahren gemäß der vorliegenden Erfindung erhalten wurden und einen erhöhten Wassergehalt aufweisen, ist ein weiterer Gegenstand der Erfindung.

Die Hydrierung wird durchgeführt unter den in der Literatur bekannten Bedingungen und mit den bekannten Materialien. Es werden dabei vorzugsweise Cuhaltige Katalysatoren verwendet. Weitere bevorzugte Katalysatoren sind in den Anmeldungen DE-A-24 45 303, DE-A-198 09 418, DE-A-197 30 939 und WO 95/32171 beschrieben. Die Hydrierungen werden durchgeführt bei Drücken von 5 bis 250 bar, vorzugsweise 10 bis 200 bar, besonders bevorzugt 20 bis 150 bar.

Die Erfindung wird nun in nachfolgenden Beispielen erläutert. In diesen Beispielen wurde ein Aldolisierungsgemisch verwendet, das wie folgt dargestellt worden war:

Eine Apparatur bestehend aus zwei beheizbaren, durch Überlaufrohre miteinander verbundenen Rührkesseln mit einem Fassungsvermögen von insgesamt 72 1 wurde mit frischer, wäßriger Formaldehydlösung (4300 g/h) in Form einer 40 %igen wäßrigen Lösung bzw. n-Butyraldehyd (1800 g/h) und mit frischem Trimethylamin als Katalysator (130 g/h) in Form einer 45 %igen wäßrigen Lösung kontinuierlich beschickt. Der Reaktoren wurden dabei auf 40°C temperiert. Es kann dabei ein methanolarmes Formaldehydgemisch verwendet werden, wie dies in der deutschen Anmeldung mit dem Titel "Verfahren zur Herstellung von Polyalkoholen mit Methanol-armem Formaldehyd". Aktenzeichen 199 63 438.6 (Anmelderin: BASF AG) beschrieben ist.

Der Austrag wurde direkt in den oberen Teil eines Fallfilmverdampfers mit aufgesetzter Kolonne (11 bar Heizdampf) geleitet und dort bei normalem Druck destillativ in ein leichtsiedendes Kopfprodukt, im wesentlichen enthaltend n-Butyraldehyd, Ethylacrolein, Formaldehyd, Wasser und Trimethylamin, und ein hochsiedendes Sumpfprodukt aufgetrennt.

Das Kopfprodukt wurde kontinuierlich kondensiert und in die oben beschriebenen Reaktoren zurückgeführt.

Das hochsiedende Sumpfprodukt aus dem Verdampfer (ca. 33,5 kg/h) wurde kontinuierlich mit frischem Trimethylamin-Katalysator (50 g/h, in Form der 45 %igen wäßrigen Lösung) versetzt und in einen beheizbaren, mit Füllkörpem versehenen Rohrreaktor mit einem Leervorlumen von 12 1 geführt. Der Reaktor war dabei auf 40°C temperiert.
Der Austrag des Nachreaktors wurde kontinuierlich in den oberen Teil einer weiteren Destillationseinrichtung, der Formaldehydabtrennung, (11 bar Heizdampf) gegeben und dort destillativ in ein leichtsiedendes Kopfprodukt, im wesentlichen enthaltend Ethylacrolein, Formaldehyd, Wasser und Trimethylamin, und ein hochsiedendes Sumpfprodukt aufgetrennt. Das leichtsiedende Kopfprodukt (27 kg/h) wurde kontinuierlich kondensiert und in den ersten Rührkessel zurückgeleitet, wohingegen das hochsiedende Sumpfprodukt gesammelt wurde.

Das so erhaltene Sumpfprodukt enthielt neben Wasser im wesentlichen Dimethylolbutyraldehyd, Formaldehyd und Spuren Monomethylolbutyraldehyd.

Die folgenden Beispiele 1 bis 4 beschreiben die bessere Abtrennung von Formaldehyd und die verminderte Hochsiederbildung, die durch die erfindungsgemäße höhere Verdünnung ermöglicht werden.

### Beispiel 1 (Vergleich)

Aus dem wie oben beschriebenen erhaltenen Aldolisierungsgemisch von Formaldehyd mit n-Butyraldehyd wird der Formaldehyd destillativ abgetrennt. Die Reaktionsmischung enthält 9,4 % Dimethylolbutanal (DMB), 11,4 % Formaldehyd sowie Wasser. Die Abtrennung erfolgt in einer Kolonne mit einem Rücklaufverhältnis von 0,33. Der Kopfdruck beträgt 1,5 bar. Bei einer Zulaufmenge von 18 kg/h kann der Formaldehyd-Gehalt im Kolonnensumpf auf 3,4 Gew.-% abgereichert werden. Dieser Kolonnensumpf wird anschließend einer im allgemeinen bekannten Hydrierung unterworfen. Danach finden sich im hydrierten Sumpf 8 % hochsiedende Acetale, wobei darunter die Summe der Dimethylolbutanal- und der Trimethylolpropanacetale verstanden wird. Weiterhin finden sich 4,3 % weitere Hochsieder.

### Beispiel 2 (Vergleich)

Es wird verfahren, wie oben unter Beispiel 1 beschrieben, wobei jedoch 1,5 kg/h flüssigen Wassers in den Zulauf gegeben werden. Dabei reduziert sich die Formaldehydkonzentration im Sumpf auf 1,79 Gew.-%. Nach der Hydrierung beträgt die Acetalkonzentration 4,3 % und die der weiteren Hochsieder 3,2 %.

### Beispiel 3 (Vergleich)

Es wird verfahren wie unter Beispiel 2 beschrieben, wobei jedoch zusätzlich 1,5 kg/h Wasser in den Kolonnensumpf eingespeist werden, so daß insgesamt 3 kg/h Wasser zugegeben werden. Dadurch reduziert sich der Formaldehydgehalt auf 0,98 Gew.-%. Nach der Hydrierung befinden sich im Sumpf 4,7 % Acetale und 4,9 % weitere Hochsieder.

### Beispiel 4

Ein wie oben beschrieben erhaltenes Aldolisierungsprodukt enthaltend 9,5 % DMB und ca. 17 Gew.-% Formaldehyd, wurde in der im Beispiel 1 beschriebenen Kolonne destillativ aufgetrennt. Der Kopfdruck betrug 2 bar und das Rücklaufverhältnis 0. Es wurden 4,5 kg/h Wasserdampf in den Sumpf eingespeist. Der Aldolisierungszulauf betrug 25,6 kg/h, der Formaldehydgehalt im Sumpf konnte derart auf 0,4 Gew.-% vermindert werden. Im hydrierten Kolonnensumpf betrug die Acetalkonzentration 1,5 % und die der weiteren Hochsieder 2,8 %.

In den nachfolgenden Beispielen 5 bis 8 wird der Effekt demonstriert, den eine höhere Verdünnung nach der vorliegenden Erfindung auf die Selektivität der Hydrierung ausübt.

### Beispiele 5 bis 8

Es wurde ein Aldolisierungsgemisch verwendet, das wie vorstehend beschrieben erhalten worden war.

Die derart erhaltenen wässrigen Lösungen wurden bei 90 bar und Temperaturen von 120°C im Hauptreaktor und 130°C im Nachreaktor in Kreislauf-/Rieselfahrweise an einem Cu/TiO₂-Katalysator, hergestellt analog Katalysator F der DE 198 09 418 und enthaltend 47 Gew.-% CuO auf TiO₂, hydriert. Die dabei verwendete Apparatur bestand aus einem 160 ml großen Hauptreaktor und einem 50 ml großen Nachreaktor, die über Standhaltungen reguliert wurden. Die Kreislaufmenge betrug 1,0 l/h.

In der nachfolgenen Tabelle bezieht sich die angegebene Belastung auf den Anteil an organischem Material im Zulauf. Es finden sich weiterhin Umsatz und Selektivität, die mit einem Einsatzmaterial bestimmt wurden, das 65 % organischen Materials bzw. 40 % organischen Materials enthielt, wobei diese letzte Lösung durch Verdünnung mit Wasser aus der 65 %igen Lösung erhalten wurde. Die Selektivität bezieht sich dabei auf die eingesetzte Menge an Dimethylolbutanal.

| **Bsp** | **Einsatzmat. [%]** | **Belastung [kg orgJL Kat.*h]** | **Umsatz [%]** | **Selektivität [%]** | **Ausbeute [%]** |
|---|---|---|---|---|---|
| 5 | 65 | 0,29 | 98,59 | 87,05 | 85,83 |
| 6 | 40 | 0,29 | 100,00 | 93,77 | 93,77 |
| 7 | 65 | 0,40 | 99,34 | 89,27 | 88,68 |
| 8 | 40 | 0,40 | 99,79 | 91,34 | 91,15 |

In den nachfolgenden Beispielen 9 und 10 wird der Effekt verdeutlicht, den eine höhere Verdünnung nach der vorliegenden Erfindung bezüglich der Erhöhung der Standzeit bzw. der Verminderung der Desaktivierung des Hydrierkatalysators ausübt.

### Beispiel 9 (Vergleichsbeispiel)

Die in Beispiel 1 erhaltene wässrige Lösung wurde bei 90 bar und 115°C (Hauptreaktortemperatur) in Kreislauf-/Rieselfahrweise hydriert. Der Katalysator wurde analog Katalysator D der DE 198 09 418 hergestellt. Er enthielt 24 % CuO, 20 % Cu und 46 % TiO₂. Die verwendete Apparatur bestand aus einem 10 m langen beheizten Reaktor NW 25, einem Druckabscheider und einer Kreislaufpumpe. Der Kreislaufdurchsatz betrug 25 l/h Flüssigkeit, der Reaktorzulauf wurde auf 4 kg/h eingestellt.

Am Tag der Inbetriebnahme konnte ein Umsatz an DMB von > 99,8 % erreicht werden. Im Verlauf von 10 Wochen, in denen der Reaktor in Dauerbetrieb gefahren wurde, sank der Umsatz auf 91 % ab.

### Beispiel 10

Es wurde verfahren wie unter Beispiel 9 beschrieben, mit einem Katalysator des gleichen Typs. Wie in Beispiel 4 beschrieben, wurde jedoch Wasser zudosiert.

Am Tag der Inbetriebnahme wurde ein Umsatz an DMB von > 99,8 % gemessen, der im Verlauf von 10 Wochen Dauerbetrieb auf lediglich 98,6 % absank.

## Patentansprüche

1. Verfahren zum destillativen Abtrennen von Formaldehyd aus Reaktionslösungen enthaltend ein methyloliertes Alkanal, das aus der Umsetzung von Formaldehyd mit einem Alkanal, das wenigstens ein acides Wasserstoffatom in α-Stellung zur Carbonylfunktion aufweist, oder der Umsetzung eines 2-Alkylacroleins oder Acrolein mit Wasser und Formaldehyd erhalten wurde, und wobei diese Umsetzung in Gegenwart katalytischer Mengen eines organischen Amins durchgeführt wurde, **dadurch gekennzeichnet, dass** das Abtrennen in einer Destillationskolonne erfolgt und dass vor und/oder während der Destillation 0,1 bis 10 Teile Wasserdampf eingespeist werden, bezogen auf den Zulaufstrom.

2. Verfahren nach Anspruch 1, **dadurch gekennzeichnet, dass** die Destillation bei Temperaturen von 50 bis 160°C, Drücken von 20 mbar bis 5 bar, vorzugsweise 1 bar bis 3 bar, sowie einem Rücklaufverhältnis von 100 bis 0, vorzugsweise 3 bis 0 betrieben wird.

3. Verfahren nach Anspruch 1 oder 2, **dadurch gekennzeichnet, dass** der Wasserdampf unterhalb des Zulaufstroms oder mit dem Zulaufstrom zugeführt wird, vorzugsweise in den Kolonnensumpf oder in den Verdampfer.

4. Verfahren nach einem der Ansprüche 1 bis 3, **dadurch gekennzeichnet, dass** die Anzahl der Trennstufen der Kolonne von 3 bis 100, vorzugsweise 5 bis 60 beträgt.

5. Verfahren nach einem der Ansprüche 1 bis 4, **dadurch gekennzeichnet, dass** die Verweilzeit in der Kolonne von 1 min bis 300 min, vorzugsweise von 10 min bis 180 min beträgt.

6. Verfahren nach einem der Ansprüche 1 bis 5, **dadurch gekennzeichnet, dass** der Kolonnen- und Verdampfersumpf konstruktiv so gestaltet werden, dass die auf den Sumpfaustrag bezogenen Verweilzeiten von 1 min bis 300 min, vorzugsweise 5 min bis 120 min, betragen.

7. Verfahren nach einem der Ansprüche 1 bis 6, **dadurch gekennzeichnet, dass** die Kolonne mit einem Verdampfer mit geringer Wandtemperatur und kleinem Flüssigkeitsinhalt, vorzugsweise einem Fallfilmverdampfer, sowie einem Kondensator mit Flüssigkeitsrückführung, vorzugsweise einem Quench, betrieben wird.

8. Verfahren zur Herstellung von Polyolen, insbesondere Triolen, **dadurch gekennzeichnet, dass** ein methyloliertes Alkanal, das gemäß dem Verfahren nach einem der Ansprüche 1 bis 7 von Formaldehyd befreit wurde, einer an sich bekannten Hydrierung unterworfen wird.

## Claims

1. A process for the removal, by distillation, of formaldehyde from reaction solutions comprising a methylolated alkanal which was obtained from the reaction of formaldehyde with an alkanal which has at least one acidic hydrogen atom α to the carbonyl function or from the reaction of a 2-alkylacrolein or acrolein with water and formaldehyde, this reaction having been carried out in the presence of catalytic amounts of an organic amine, wherein the removal is effected in a distillation column and from 0.1 to 10 parts of steam are fed in before and/or during the distillation, based on the feed stream.

2. The process according to claim 1, wherein the distillation is operated at from 50 to 160°C, from 20 mbar to 5 bar, preferably from 1 to 3 bar, and a reflux ratio of from 100 to 0, preferably from 3 to 0.

3. The process according to claim 1 or 2, wherein the steam is fed in below the feed stream or with the feed stream, preferably into the bottom of the column or into the evaporator.

4. The process according to any of claims 1 to 3, wherein the number of theoretical plates of the column is from 3 to 100, preferably from 5 to 60.

5. The process according to any of claims 1 to 4, wherein the residence time in the column is from 1 to 300, preferably from 10 to 180, minutes.

6. The process according to any of claims 1 to 5, wherein the bottom of the column and the bottom of the evaporator are designed in such a way that the residence times are from 1 to 300, preferably from 5 to 120, minutes, based on the bottom discharge.

7. The process according to any of claims 1 to 6, wherein the column is operated with an evaporator having a low wall temperature and small liquid content, preferably a falling-film evaporator, and a condenser with liquid recycling, preferably a quench.

8. A process for the preparation of polyols, in particular triols, wherein a methylolated alkanal which was freed from formaldehyde by the process according to any of claims 1 to 7 is subjected to a hydrogenation known per se.

## Revendications

1. Procédé de séparation par distillation de formaldéhyde contenu dans des solutions réactionnelles contenant un alcanal méthylolé, obtenu par réaction de formaldéhyde avec un alcanal présentant au moins un atome d'hydrogène acide en position α par rapport à la fonction carbonyle, ou par réaction d'une 2-alkylacroléine ou d'acroléine avec de l'eau et du formaldéhyde, cette réaction ayant été entreprise en présence de quantités catalytiques d'une amine organique, **caractérisé en ce que** la séparation a lieu dans une colonne de distillation et **en ce que** l'on alimente, avant et/ou pendant la distillation, de 0,1 à 10 parties de vapeur d'eau, par rapport au courant d'amenée.

2. Procédé suivant la revendication 1, **caractérisé en ce que** la distillation est entreprise à des températures de 50 à 160°C, des pressions de 20 mbar à 5 bar, de préférence de 1 bar à 3 bar, et avec une proportion de reflux de 100 à 0, de préférence de 3 à 0.

3. Procédé suivant la revendication 1 ou 2, **caractérisé en ce que** la vapeur d'eau est alimentée au-dessous du courant d'amenée ou avec le courant d'amenée, de préférence dans le fond de la colonne ou dans l'évaporateur.

4. Procédé suivant l'une quelconque des revendications 1 à 3, **caractérisé en ce que** le nombre des plateaux de la colonne est de 3 à 100, de préférence de 5 à 60.

5. Procédé suivant l'une quelconque des revendications 1 à 4, **caractérisé en ce que** le temps de séjour dans la colonne est de 1 minute à 300 minutes, de préférence de 10 minutes à 180 minutes.

6. Procédé suivant l'une quelconque des revendications 1 à 5, **caractérisé en ce que** le fond de la colonne et de l'évaporateur est construit de manière à ce que les temps de séjour, ramenés à la sortie de fond, soient de 1 minute à 300 minutes, de préférence de 5 minutes à 120 minutes.

7. Procédé suivant l'une quelconque des revendications 1 à 6, **caractérisé en ce que** la colonne fonctionne avec un évaporateur à basse température de paroi et faible contenu' de liquide, de préférence un évaporateur à film tombant, ainsi qu'un condenseur à retour de liquide, de préférence un refroidisseur rapide.

8. Procédé de préparation de polyols, en particulier de triols, **caractérisé en ce que** l'on soumet un alcanal méthylolé, débarrassé de formaldéhyde par le procédé suivant l'une quelconque des revendications 1 à 7, à une hydrogénation connue en soi.
